# EUROPEAN PATENT APPLICATION

(11) **EP 1 323 987 A2**
(43) Date of publication of application: **02.07.2003**
(21) Application number: 02080092.6
(22) Date of filing: 06.12.2002
(51) Int. Cl.: F24F 3/16

(54) **Air treatment arrangement**

(30) Priority: 19.12.2001 NL 1019603
(71) Applicant: J.A. Niemeijer B.V., 7591 DB Denekamp (NL)
(72) Inventor: Niemeijer, Johannes Antonius, Deceased (NL)

(57) **Abstract**

The invention relates to a rotating air treatment arrangement, for cleaning or cooling air. The rotating air treatment arrangement consists of a cylindrically shaped packing material (11,15,17,20), made of a synthetic material, which rotates and which is under operational condition partly placed in a rinsing liquid (12). The air to be cleaned is guided through the packing material (11,15,17,20), which is constantly kept wet in this way.

## Description

The invention relates to an air treatment arrangement, for cleaning and/or cooling air, comprising a washing section provided with a packing material having a large internal surface area, which is moisturised with a liquid and through which air to be treated is passed.

Air treatment arrangements of this type are known. They are frequently used, for example in the bio-industry, but also in plants in which for example refuse or manure is processed. In the known arrangements, the air to be cleaned or cooled is blown through the packing material, while water is distributed over the packing material with the aid of a number of nozzles. Subsequently, the water is freed from picked-up dust particles and ammonia in a biological cleaning process or the water is poured in a well if it concerns a cooling process.

The problem of the known air treatment arrangements is that they have a limited capacity per unit of volume. Moreover, circulating the washing water costs relatively much energy. The air treatment arrangement according to the invention substantially obviates these drawbacks and is characterised in that the packing material is mounted for rotation round a longitudinal axis, that the packing material is operationally at least partly positioned in a bath, filled with liquid and that drive means are provided for operationally rotating the packing material round its longitudinal axis. In this way, the liquid on the surface of the packing material is continuously refreshed, with a minimal consumption of energy.

A favourable embodiment is characterised in that the packing material comprises a set of discs, mounted round the longitudinal axis, between which the air to be treated can pass under operational conditions. A packing material of this kind can be fabricated in a simple manner and the discs may be very thin, which means that a large effective surface area can be realised within a limited volume. It is also possible to operate a packing material of this kind at a relatively high number of revolutions, in which case the turbulence at the surface of the discs provides for a good interaction between the moistened surface of the discs and the air passing between them. Subjecting the discs to a surface treatment process, in order to obtain a larger effective surface area may increase the effectiveness even further.

An alternative favourable embodiment is characterised in that the packing material comprises an at least substantially cylindrical body, of which an axis of symmetry at least substantially coincides with the longitudinal axis and that the packing material has an open-worked structure, through which air to be treated can pass under operational conditions. An air treatment arrangement of this kind is preferably operated at a relatively low number of revolutions, thanks to which the consumption of energy is lower. The washing capacity remains sufficient, because the air must flow now through the packing material, which produces much turbulence so that still a good interaction with the moistened surface is obtained. In this embodiment, the packing material is preferably made of a synthetic material, which keeps the weight low and which results in an arrangement that can be used substantially maintenance free.

An alternative favourable embodiment is characterised in that the packing material comprises an at least substantially cylindrically shaped basket, of which an axis of symmetry at least substantially coincides with the longitudinal axis and that the basket is filled with objects that have an open-worked structure, through which air to be treated can pass under operational conditions. In this embodiment, the basket is for example made of a stainless steel wire mesh, which results in a basket having an acceptable weight and which still is sufficiently strong. The objects are preferably made of a synthetic material or stainless steel or a ceramic material.

A further favourable embodiment of the inventive air treatment arrangement is characterised in that the liquid consists at least substantially of water. This embodiment of the air treatment arrangement may be part of for example a biological cleaning arrangement, in which the packing material may actively participate in the biological process in which ammonia is converted into nitrates and nitrites in which subsequently the washing water is cleaned further with the aid of biological processes, in such a way that it can be reused over and over again. Also when the air treatment arrangement forms part of a cooling arrangement, water is preferably used. The water may be pumped out of a well, while the heated up water is poured in another well.

A favourable alternative embodiment is characterised in that the liquid consists at least substantially of acidified water. This embodiment should be given preference in case extreme quantities of ammonia are produced. In order not to interfere with the biological processes, a biological air treatment arrangement would need to have an enormous volume. In this embodiment, the ammonia is not converted in a biological process, but in a chemical process that is known to be very robust and efficient.

In a favourable embodiment, the acidified water has a pH of 2-5, a value that can be obtained easily and that is sufficient to remove the ammonia substantially completely. In a further favourable embodiment, the air treatment arrangement is provided with a droplet collector, mounted inside an exhaust channel, which prevents the acidified water or water containing ammonia from spreading outside the air treatment arrangement.

A further favourable embodiment of the inventive air treatment arrangement is characterised in that it moreover comprises a prewash section, for removing dust particles. This prevents the rotating washer from becoming less effective after some time, because of a deposit of dust particles.

The invention will now be further explained with a reference to the following figures, in which:
- Fig. 1: represents in a block diagram a possible embodiment of the air treatment arrangement;
- Fig. 2: schematically represents in top view a possible embodiment of a washing section;
- Fig. 3: schematically represents this embodiment in side view;
- Fig. 4: schematically represents in side view a possible embodiment of a cooling arrangement;
- Fig. 5A: represents more in detail a packing material built-up of discs;
- Fig. 5B: represents more in detail a packing material consisting of cylindrical subbodies;
- Fig. 5C: represents more in detail a packing material consisting of a cylindrically shaped basket;
- Fig. 6: represents a possible embodiment of a laminated thickener.

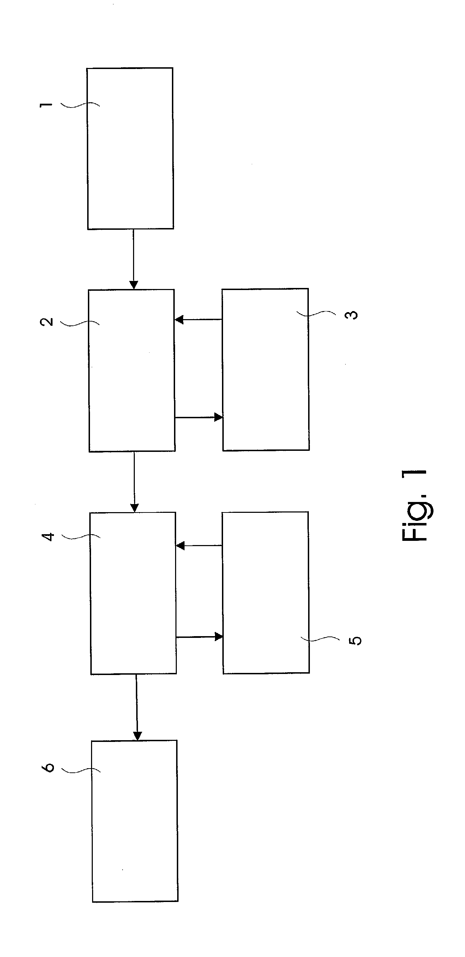

Fig. 1 represents in a block diagram a possible embodiment of the air treatment arrangement which is used as an air washer, consisting of a ventilator 1 which blows polluted air through the air treatment arrangement, which air is first guided through a prewash section 2 in which dust particles are to a large extent removed. The particles and the washing water are guided to a laminated thickener 3 or to a filter arrangement and the cleaned-up water lead back to prewash section 2. The air, which is free now from dust particles, is guided to a rotating air treatment arrangement 4, in which ammonia, present in the air, is converted to a salt and subsequently carried off to a conditioner 5. Rotating air treatment arrangement 4 may be operated with water, in which case conditioner 5 comprises a denitrification unit, well known in the art, which produces clean water that can be reused in rotating air treatment arrangement 4. Rotating air treatment arrangement 4 may also be operated with acidified water, by adding for example sulphuric acid to the water. In that case, conditioner 5 comprises a discharge vessel, in which water with ammonium sulphate dissolved in it can be collected and a supply vessel in which a fresh sulphuric acid solution is stored that can periodically be supplied to rotating air treatment arrangement 4. The air treatment arrangement finally comprises a droplet collector 6, which prevents water with salts or acid dissolved in it form being spread in the atmosphere.

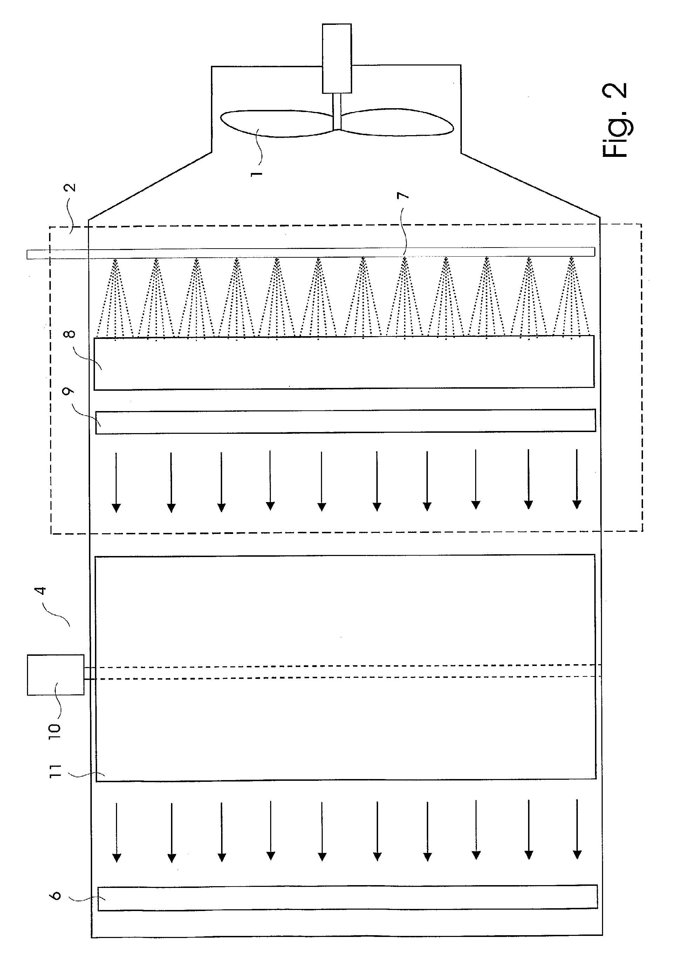

Fig. 2 schematically represents in top view a possible embodiment of a washing section, consisting of a ventilator 1, a prewash section 2, a rotating air treatment arrangement 4 and a droplet collector 6. Prewash section 2 comprises a packing material 8 with an open structure, as such known in the art, onto which water is sprayed with the aid of nozzles 7 and through which air to be cleaned is guided. In this process, dust particles collect in packing material 8 and the dust leaves the prewash section together with the water. Behind packing material 8, a droplet collector 9, well known in the art, is placed, in order to prevent the water to enter the rotating air treatment arrangement 4. The air, cleaned in this manner is supplied to rotating air treatment unit 4, which is driven by a motor 10 and in which a cylindrically shaped packing material 11 rotates partly inside a tank, filled with a liquid (not visible in this figure). Cylindrically shaped packing material 11 has an open structure, through which the air to be cleaned can flow and in the process of which the ammonia present in the air may react with the cleaning liquid, taken along by the packing material 11. Cleaned air is subsequently guided through a droplet collector 6, well known in the art.

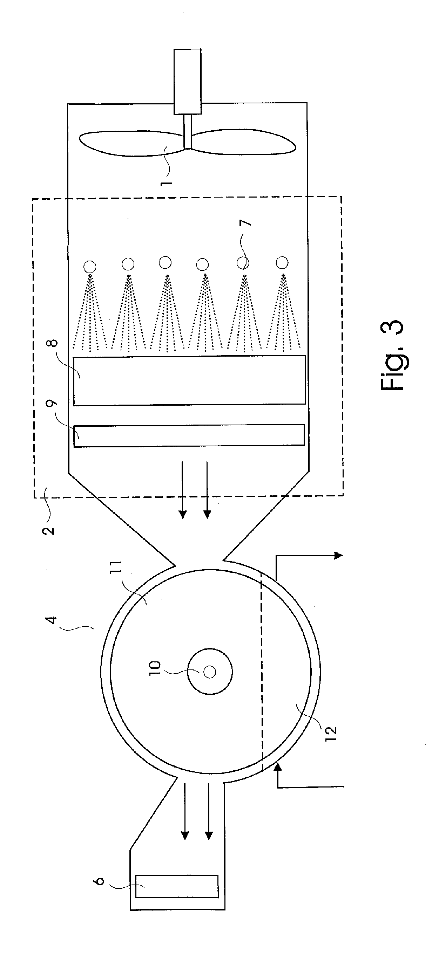

Fig. 3 schematically represents this embodiment in side view, with ventilator 1, prewash section 2, rotating air treatment arrangement 4 and droplet collector 6. Behind packing material 8, a droplet collector 9 is placed, well known in the art, in order to prevent water to enter rotating air treatment arrangement 4. The cleaned air is taken back to rotating air treatment arrangement 4, which is driven by a motor 10 and of which a cylindrically shaped packing material 11 rotates partly in a tank, filled with a liquid 12. Cleaned air is subsequently guided through a droplet collector 6, well known in the art.

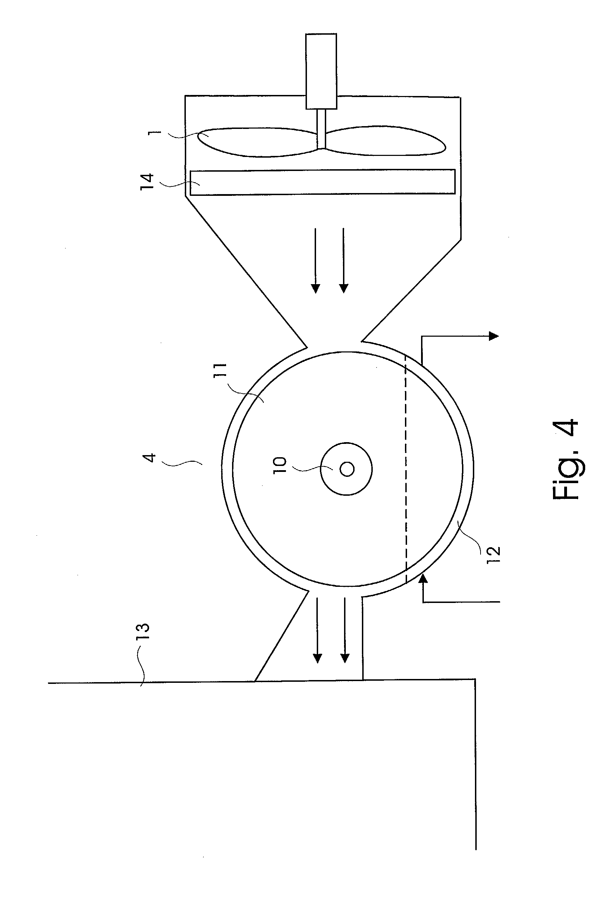

Fig. 4 schematically represents in side view a possible embodiment of the air treatment arrangement used as a cooling arrangement, for example for cooling air supplied to a shed or sty 13. In this embodiment, the arrangement is provided with a ventilator 1, a simple dust filter 14 and a rotating air treatment arrangement 4. Filtered air is supplied from outside to a rotating air treatment arrangement 4, driven by a motor 10 of which a cylindrically shaped packing material 11 rotates partly in a tank, filled with water 12. Subsequently, the cooled air is blown directly into shed or sty 13. Water 12 may be taken for example from a well, while the heated-up water is pumped into another well. By utilising natural draught, the arrangement may be operated without a ventilator. One may for example mount chimneys on top of shed or sty 13 and make the warm air flow through it. This will build up a slight underpressure in shed or sty 13, which is sufficient to make the air flow through arrangement 4.

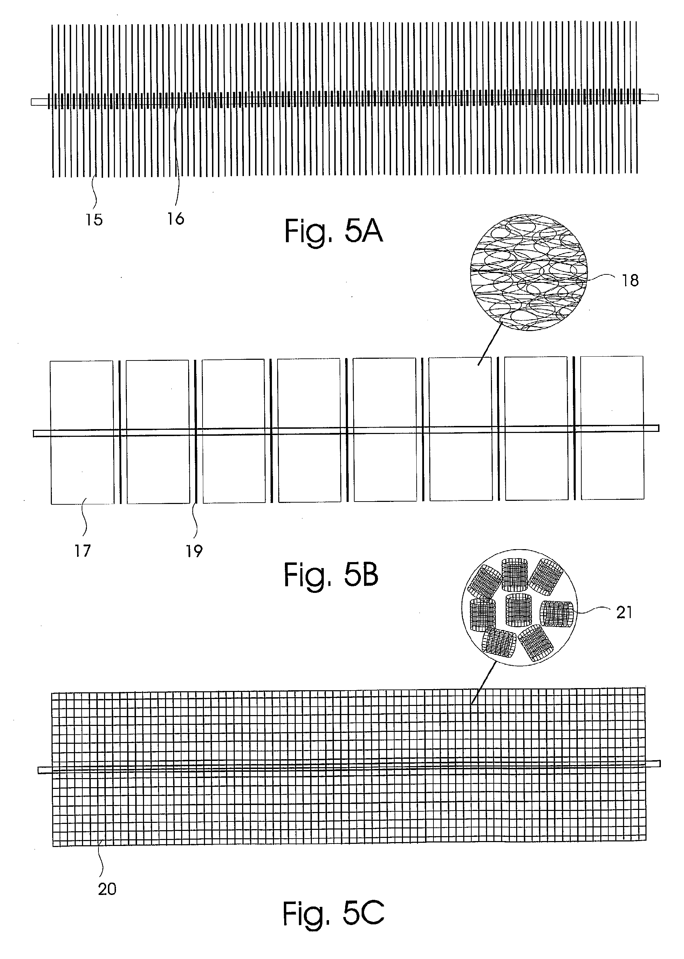

Fig. 5A represents more in detail a packing material 11 built-up of discs 15. Discs 15 are preferably made of a synthetic foil, for example polyethylene and have a thickness of for example 0.5-mm. Mutually, a distance of for example 1-mm is maintained with the aid of intermediate discs 16. A packing material of this kind passes the air unhindered, while it may have a surface area of hundreds of square meters on which ammonia gas may react with the liquid.

Fig. 5B represents more in detail a packing material consisting of cylindrical subbodies 17, which subbodies consist of fine three-dimensional bodies made of synthetic wire which are mutually welded together, shaped for example as schematically shown in the inset 18. This kind of structures combines a large surface area with a good permeability to air. The structures may be obtained for example shaped as matting, of which discs can be cut which are subsequently combined to a cylindrically shaped body. If desired, plates 19 may be included between the subbodies, for increasing the strength of the cylindrically shaped body.

Fig. 5C represents more in detail a packing material consisting of a cylindrically shaped basket 20, manufactured of stainless steel wire mesh, which is filled with objects that have an open-work structure, such that under operational conditions the air to be cleaned or cooled can flow through it. The objects are made of a corrosion resistant material, for example plastic or stainless steel or a ceramic material and they may have simple structures that can be manufactured cheap and in large volumes, like for example the open-work cylinders shown in inset 21, which bodies comprise a large number of ribs.

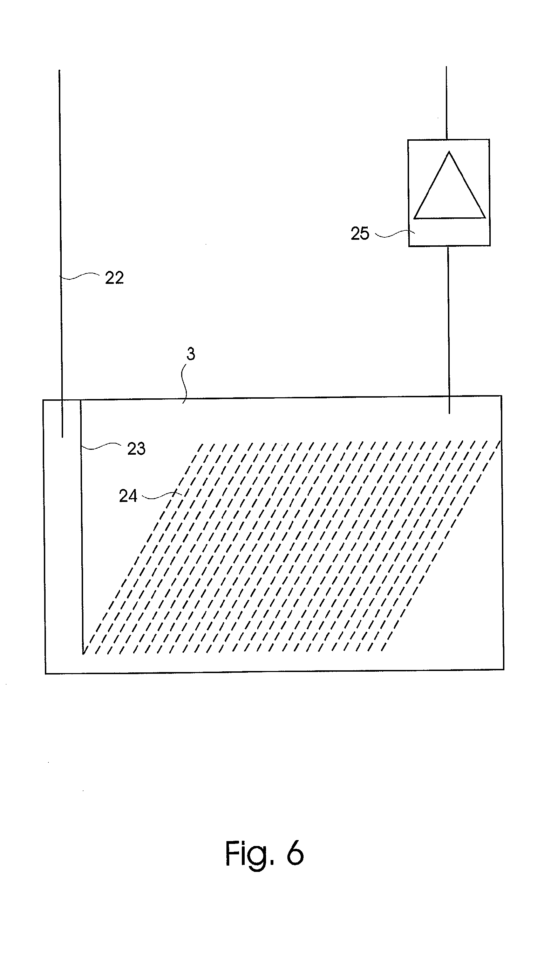

Fig. 6 represents a possible embodiment of a laminated thickener 3, which receives water originating from prewash section 2. The water flows via entry 22 and a partition 23 towards the bottom, then upwards through a slantingly positioned packing material 24, built up of strips or tubes, and subsequently it is pumped back to prewash section 2 by a pump 25. When the water flows upwards, dust and dirt will be deposited shaped as a layer onto the surface of the packing material 24. If the thickness of the layer has become sufficiently large, it will peel off under the influence of gravity and sink to the bottom of laminated thickener 3. This bottom can easily be cleaned periodically.

## Claims

1. Air treatment arrangement, for cleaning and/or cooling air, comprising a washing section provided with a packing material having a large internal surface area, which is moisturised with a liquid and through which air to be treated is passed, **characterised in that** the packing material mounted for rotation round a longitudinal axis, that the packing material is operationally positioned at least partly in a bath, filled with liquid and that drive means are provided for operationally rotating the packing material round its longitudinal axis.

2. Air treatment arrangement according to claim 1, **characterised in that** the packing material comprises a set of discs, mounted round the longitudinal axis, between which the air to be treated can pass under operational conditions.

3. Air treatment arrangement according to claim 1, **characterised in that** the packing material comprises an at least substantially cylindrical body, of which an axis of symmetry at least substantially coincides with the longitudinal axis and that the packing material has an open-worked structure, through which air to be treated can pass under operational conditions.

4. Air treatment arrangement according to claim 3 of 4, **characterised in that** the packing material is made of a synthetic material.

5. Air treatment arrangement according to claim 1, **characterised in that** the packing material comprises an at least substantially cylindrically shaped basket, of which an axis of symmetry at least substantially coincides with the longitudinal axis and that the basket is filled with objects that have an open-worked structure, through which air to be treated can pass under operational conditions.

6. Air treatment arrangement according to one of the previous claims, **characterised in that** the liquid consists at least substantially of water.

7. Air treatment arrangement according to claim 3 or 4, **characterised in that** the liquid consists at least substantially of acidified water.

8. Air treatment arrangement according to claim 7, **characterised in that** the pH-value of the acidified water amounts to 2-5.

9. Air treatment arrangement according to claim 7 or 8, **characterised in that** the air treatment arrangement is provided with a droplet collector, mounted inside an exhaust channel.

10. Air treatment arrangement according to claim 6 or 7, **characterised in that** the air treatment arrangement moreover comprises a prewash section, for removing dust particles.
